# EUROPEAN PATENT APPLICATION

(11) **EP 3 957 369 A1**
(43) Date of publication of application: **23.02.2022**
(21) Application number: 20192039.4
(22) Date of filing: 20.08.2020
(51) Int. Cl.: A62B 7/08, A61M 16/06, A61M 16/22, A62B 7/10, A62B 9/06, A62B 18/02, A62B 18/08, A62B 21/00, A62B 23/02

(54) **BREATHING MASK WITH FILTER**

(71) Applicant: Olero IP AB, 172 66 Sundbyberg (SE)
(72) Inventor: BERZELIUS, Jonny, 111 27 Stockholm (SE); TELBY, Ola, 652 25 Karlstad (SE)
(74) Representative: AWA Sweden AB

(57) **Abstract**

A breathing mask (100) comprising at least one filter (120) configured to remove contaminants from gas passing through the at least one filter is provided. The breathing mask comprises a filter housing (110) arranged to house the at least one filter. The breathing mask further comprises a face piece (130) for arrangement on a user's face, wherein the face piece at least partially encloses the filter housing. The filter housing comprises a port (112) oppositely arranged the face piece, and at least one aperture (114) opening into the face piece. The filter housing allows for gas to pass between the port and the at least one aperture through the at least one filter.

## Description

### FIELD OF THE INVENTION

The present invention generally relates to breathing masks. More specifically, the present invention relates to a breathing mask comprising a filter.

### BACKGROUND OF THE INVENTION

Breathing masks have many uses in numerous fields. They can be used to protect a user from harmful particles, gases or organisms that can exist in the ambient air. People who work in environments where they are exposed to contaminants, such as firemen, may use breathing masks to keep them safe while working. Breathing masks can be used by medical staff or patients to protect themselves and others from e.g. bacterial and viral organisms. Further examples of medical arrangements which may use breathing masks include respiratory systems and other breathing devices.

Due to an increase of air pollution and harmful particles and organisms in today's society, and the health problems associated with air pollution, there is a high interest in the development of breathing masks. In particular, there is a high demand of efficient breathing masks that furthermore are versatile and comfortable for the user.

In the prior art, there are numerous breathing masks with different designs, filters, etc., wherein these breathing masks may be adapted for specific purposes.

US2018296864A discloses a breathing mask/respirator with a face mask and filter that extends around the neck. US2019321663A discloses a breathing mask/respirator with face mask and a filter, with a replaceable filtration system to prevent inhalation of e.g. dangerous fumes. US2019358473A discloses a breathing mask with a filter, for filtration of airborne particles. US20120174922A1 discloses a breathing device using a mask, a flow generator and a filter. US2007095348A discloses a breathing mask where part of the mask consists of filtering material. US2013139816A discloses a full-face breathing mask with a chin collar containing a filter, a bio-degradable filter. EP3485944A1, which discloses a breathing mask, with a CO₂ filter and a particle filter, allows part of the exhaled air to be recirculated.

However, one of the problems with the breathing masks of the prior art is that they are not able to provide an adequate filtering efficiency whilst being conveniently portable and/or comfortable for a user.

Hence, it is an object of the present invention to overcome these issues and to provide a breathing mask with efficient filtering properties whilst being conveniently portable and comfortable for a user, in order to provide a user-friendly and versatile alternative to the breathing masks of the prior art.

### SUMMARY OF THE INVENTION

Thus, it is of interest to explore the possibility of providing a breathing mask with efficient filtering properties whilst being conveniently portable and comfortable for a user.

This and other objects are achieved by providing a breathing mask having the features in the independent claim. Preferred embodiments are defined in the dependent claims.

Hence, according to the present invention, there is provided a breathing mask. The breathing mask comprises at least one filter configured to remove contaminants from gas passing through the at least one filter. The breathing mask further comprises a face piece for arrangement on a user's face, wherein the face piece at least partially encloses the filter housing. The filter housing comprises a port oppositely arranged the face piece, and at least one aperture opening into the face piece, wherein the filter housing allows for gas to pass between the port and the at least one aperture through the at least one filter.

Thus, the present invention is based on the idea of providing a breathing mask with a filter integrated in a face piece of the breathing mask. It will be appreciated that due to the filter housing being enclosed in the face piece, the breathing mask is easier and more comfortable to wear compared to breathing masks in the prior art. More specifically, the construction of the breathing mask results in a relatively low torque, since the weight of the breathing mask and filter is close to a user's face. It will be further appreciated that the amount of dead space is reduced by the filter being integrated in the face piece of the breathing mask according to the present invention. It should be noted that a mask having a relatively large dead space may consequently require a relatively large lung volume and/or lung capacity of the user. In contrast, by the relatively small amount of dead space in the breathing mask of the present invention, it is easier for a user to breathe, and in particular in case of a user having a reduced lung volume and/or lung capacity. By the term "dead space" it is here meant, but not limited to, the space that the inhalation or exhalation air needs to flow through before reaching a space in which gas exchange may occur. The gas being exchanged may be oxygen and/or carbon dioxide. The air may be exchanged in e.g. a user's lungs, outside air, or in a breathing device such as a rebreather connected to the breathing mask. Furthermore, the breathing mask of the present invention is relatively compact, and is therefore convenient to use and/or to store. Furthermore, the breathing mask of the present invention is advantageous in that it provides good vision for a user due to its compactness.

It will be appreciated that the breathing mask of the present invention may be used in many different fields and for many different uses. For example, the breathing mask may be used for medical patients. Notably, the breathing mask is particularly advantageous for medical patients that are lying down, e.g. in a hospital bed, as the breathing mask is comfortable to use whilst providing an efficient filtering. The breathing mask may be conveniently used in conjunction with medical equipment, such as a respirator. Further, the breathing mask may be used by people working in various fields, e.g. firemen, militaries or medical personnel. The breathing mask comprises at least one filter. The one or more filters is configured to remove contaminants from gas passing through the at least one filter. By the term "contaminants" it is here meant a constituent, impurity or substantially any other element or composition. For example, the contaminants may be particles, chemical substances, bacteria and/or virus that may be harmful for living creatures. It is to be understood that the breathing mask may comprise one single filter, or several filters patched together, or several filters arranged separately.

The breathing masks further comprises a filter housing arranged to house the at least one filter. By the term "house", it is here meant that the filter housing is arranged or configured to house, enclose or accommodate the filter(s). The filter housing may hold the at least one filter in place. The filter housing may create a sealed connection between the filter and the filter housing, such that the gas which passes through the breathing mask must pass through the at least one filter.

The breathing mask further comprises a face piece for arrangement on a user's face. The face piece at least partially encloses the filter housing. By the term "face piece" it is here meant the part of the breathing mask which is arranged to come into contact with the user's face. It will be appreciated that the face piece of the breathing mask may form a relatively tight seal with a user's face when the breathing mask is being used. It is to be understood that the face piece may have different designs and appearances, and should not limit the scope of the breathing mask.

Furthermore, the filter housing comprises a port oppositely arranged the face piece. It will be appreciated that the filter housing port is also referred to as the port of the filter housing. By the term "port" it is here meant an opening or hole with substantially any cross-sectional shape. For example, the port may be shaped as a tube. The port may also be configured to engage with an external element, for example a duct, pipe, tube or another port. The filter housing further comprises at least one aperture opening into the face piece, wherein the housing allows for gas to pass between the port and the at least one aperture through the at least one gas filter.

According to an embodiment of the present invention, the filter housing is releasably attached to the face piece. The present embodiment is advantageous in that the filter housing may be replaced easily. It will be appreciated that since the filter housing contains the filter, also the replacing of the filter(s) is made easier. This is further advantageous in that after usage, the filter(s) may be conveniently exchanged such that a new (non-contaminated) filter may be used for another person using the breathing mask. Consequently, no sterilization of the port of the filter housing is needed.

According to an embodiment of the present invention, the face piece of the breathing mask comprises an opening. The opening of the face piece may be at least partially flexible, and the port of the filter housing may be rigid, wherein the filter housing is releasably attached to the face piece by the port of the filter housing being fittingly inserted into the opening of the face piece. It is to be understood that the cross section of the port is at least slightly larger than the opening of the face piece, wherein the opening of the face piece is flexible enough to be deformed when the port is inserted. When the port of the filter housing is inserted into the opening of the face piece, the opening is deformed enough so that the port may be inserted. By this arrangement, the port is prevented from sliding out from the opening of the face piece. It will be appreciated that the present embodiment allows for an easy connection between the face piece and the filter housing.

According to an embodiment of the present invention, the at least one filter comprises a bio-filter. By the term "bio-filter" it is here meant a filter capable of filtering biological elements, such as bacteria or virus. The bio-filter may be a fine particulate filter or an adsorption filter, or a combination of both. It will be appreciated that the filter may be used to avoid ingress of biological material, such as bacteria or virus or vomit from a person. The filter may be a high efficiency particulate arresting (HEPA) filter.

According to an embodiment of the present invention, the face piece comprises a cushion part arranged to come in contact with a user's face. The present embodiment is advantageous in that the face piece can create an improved seal with a user's face. The present embodiment is further advantageous by an increased comfort when wearing the breathing mask.

According to an embodiment of the present invention, the cushion part is inflatable, wherein the face piece comprises a valve arranged externally on the cushion part for inflation of the cushion part. This is advantageous in that the cushion part is easily inflated and re-inflated when needed.

According to an embodiment of the present invention, the filter housing comprises a first part and a second part, wherein the first and second part are configured to hold the filter in place between them. It will be appreciated that the present construction of the breathing mask comprises two parts to hold the filter, which allows for an easier manufacture.

According to an embodiment of the present invention, the at least one aperture comprises a plurality of apertures. This is advantageous in that a relatively high air flow through the filter is possible, while at the same time having sufficient support for the filter. Hence, the present embodiment is particularly advantageous for a user breathing heavily and/or intense, e.g. due to physical effort.

According to an embodiment of the present invention, the breathing mask comprises a sealing ring, arranged between the port of the filter housing and the face piece. This is advantageous in that it creates a tighter seal between face piece and the filter housing.

According to an embodiment of the present invention, there is provided a breathing device. The breathing device comprises a breathing mask according to any one of the preceding embodiments. The breathing device further comprises a rebreather for closed rebreathing, wherein the rebreather comprises a counter lung and a valve housing connected to the counter lung, wherein the port of the filter housing is releasably connectable to a port of the valve housing. By the term "rebreather", it is here meant a breathing apparatus that absorbs carbon dioxide from a user's exhaled breath, allowing the exhaled air to be rebreathed (recycled) by the user. The rebreather may comprise means for adding extra oxygen, to replenish the amount metabolised by the user. By the term "counter lung", it is here meant a component, used in for example a rebreather, which changes in volume by the same amount as the user's lung volume changes. The counter lung allows the volume of the rebreather system to alternate when the user breathes, thereby letting the total volume of gas in the rebreather and in the lungs to remain the same. It will be appreciated that the breathing device according to the present embodiment allows for a more comfortable breathing device which furthermore is conveniently portable. It will be appreciated that one reason for the improved portability and comfort of the breathing device is the compact design of the breathing mask, which allows the center of mass of the breathing device be close to a user's face, thus reducing the torque effect.

According to an embodiment of the present invention, the face piece of the breathing mask of the breathing device is arranged to at least partially enclose the port of the filter housing and the port of the valve housing in a sealing manner. This is advantageous in that the sealing is improved.

According to an embodiment of the present invention, the breathing device further comprises a coupling mechanism configured to couple the port of the filter housing and the port of the valve housing. This is advantageous in that the breathing mask and the rebreather are connected, and are not unintentionally separated.

Further objectives of, features of, and advantages with, the present invention will become apparent when studying the following detailed disclosure, the drawings and the appended claims. Those skilled in the art will realize that different features of the present invention can be combined to create embodiments other than those described in the following.

### BRIEF DESCRIPTION OF THE DRAWINGS

This and other aspects of the present invention will now be described in more detail, with reference to the appended drawings showing embodiment(s) of the invention.
Figs. 1 and 2 schematically shows cross-sectional side views of a breathing mask according to exemplifying embodiments of the present invention.
Fig. 3 schematically shows a view of a filter housing according to an exemplifying embodiment of the present invention.
Fig. 4 schematically shows a view of a breathing device comprising a breathing mask and a rebreather.

### DETAILED DESCRIPTION

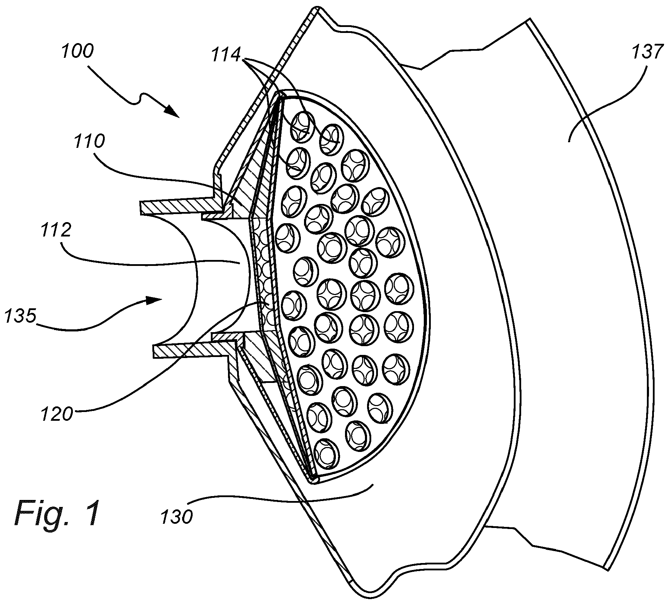

Fig. 1 schematically shows a cross-sectional side view of a breathing mask 100, according to an exemplifying embodiment of the present invention. The breathing mask 100 comprises at least one filter 120 configured to remove contaminants from gas passing through the at least one filter. The breathing mask 100 further comprises a filter housing 110 arranged to house the at least one filter 120. It is to be understood that the filter housing 110 may have different shapes, sizes and/or dimensions. The breathing mask 100 further comprises a face piece 130 for arrangement on a user's face, wherein the face piece 130 at least partially encloses the filter housing 110. The filter housing 110 comprises a port 112 oppositely arranged the face piece 130. The filter housing 110 comprises at least one aperture 114 opening into the face piece 130, wherein the filter housing 110 allows for gas to pass between the port 112 and the at least one aperture 114 through the at least one filter 120. The at least one aperture 114 in Fig. 1 comprises a plurality of apertures 114. It is to be understood that the aperture(s) 114 may have different shapes and sizes.

In Fig. 1, the face piece 130 comprises a cushion part 137 arranged to come in contact with a user's face. The face piece 130 may for example be made in a flexible rubber material, like silicone rubber. The cushion part 137 may be inflatable. The cushion part 137 may comprise e.g. plastic or rubber. The cushion part 137 is configured to create a seal with a user's face. The cushion part 137 may have a partly tubular shape that extends around the perimeter of the part of the face piece 130 which is arranged to come in contact with a user's face.

Further, in Fig. 1, the filter housing 110 comprises a first part 310 which may comprise the port 112, and a second part 320 which may comprise the at least one aperture 114. The first part and the second part 310, 320 of the filter housing 110 are configured to hold the filter(s) 120 in place between them.

Further, in Fig. 1, the breathing mask 100 comprises an opening 135. The opening 135 may be shaped in the form of a tube, with one first opening that exits the breathing mask 100 away from the user and with one second opening that is configured to receive the port 112. The part of the breathing mask 100 which constitutes the opening 135 may be at least partly flexible, and the port 112 of the filter housing 110 may be rigid, allowing the port 112 of the filter housing 110 to be inserted into the opening 135. The port 112 may have an annular shape and the radius of the tube shape of the opening 135 in a resting state, i.e. when it is not stretched or deformed by an external force, may be smaller than the outer radius of the port 112, which allows the filter housing 110 to be attached to the face piece 130 by friction between the port 112 and the opening 135.

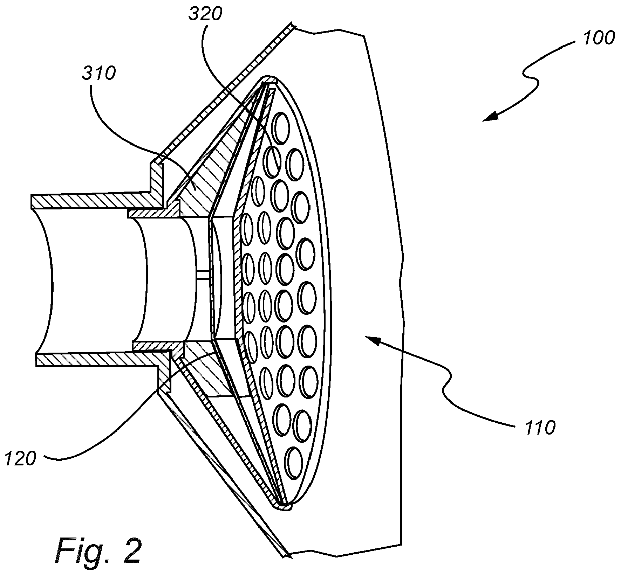

Fig. 2 schematically shows a cross-sectional side view of a breathing mask 100, according to an exemplifying embodiment of the present invention. In Fig. 2, the filter housing 110 comprises a first part 310 and a second part 320. The first part 310 and the second part 320 are configured to hold a filter 120 in place between them.

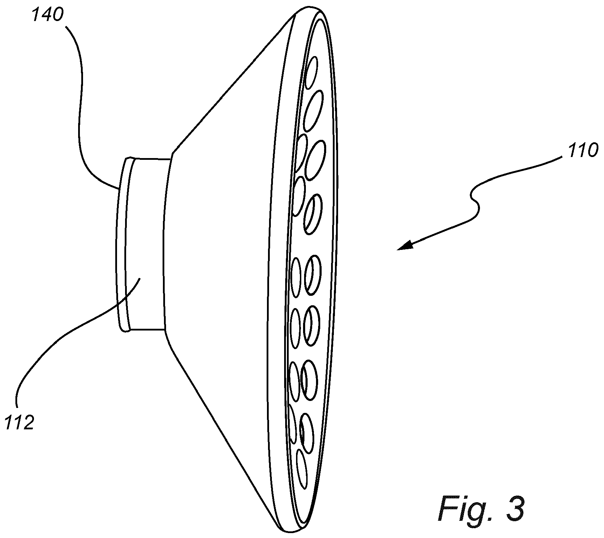

Fig. 3 shows a view of a filter housing 110 according to an exemplifying embodiment of the present invention. The filter housing 110 comprises a sealing ring 140 arranged on the port 112 of the filter housing 110, wherein the sealing ring 140 is arranged to create a tight seal between the port 112 of the filter housing 110 and the face piece (not shown). The sealing ring 140 may be arranged between the port 112 of the filter housing 110 and the part of the face piece (not shown) which constitutes the opening (not shown).

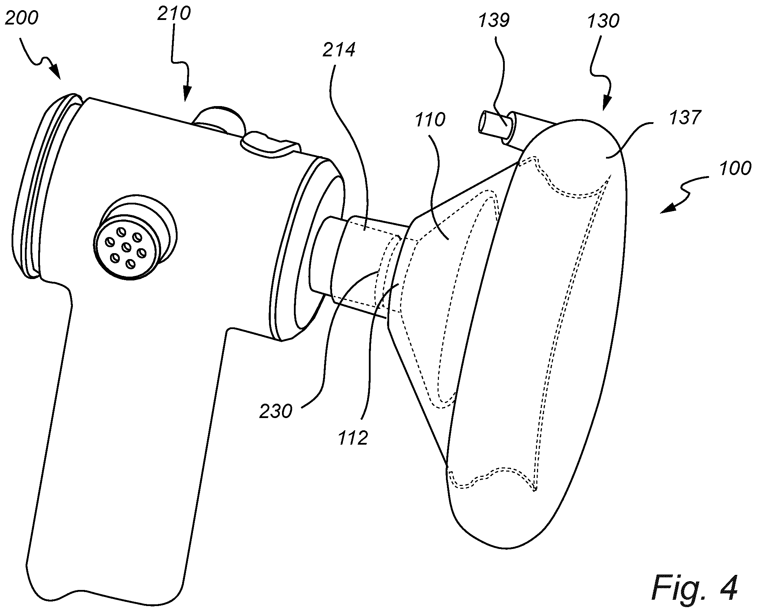

Fig. 4 schematically shows a view of a breathing device 200 comprising a breathing mask 100 according to any embodiment of the present invention and a rebreather 210. The breathing mask 100 comprises a filter housing 110. The filter housing 110 is arranged to be inserted into a port of the valve housing 214 of the rebreather 210. The breathing mask 100 further comprises a face piece 130, wherein the face piece 130 comprises a cushion part 137 and a valve 139. The valve 139 may be arranged externally on the cushion part 137 for inflation of the cushion part 137. In Fig. 4, the face piece 130 is arranged to at least partially envelop or enclose the port 112 of the filter housing 110 and the port of the valve housing 114 of the rebreather 210, wherein the envelopment provides both a tight seal and a strong connection between the breathing mask 100 and the rebreather 210. The rebreather 200 is a closed re-breathing system and may comprise a carbon dioxide scrubber for the exhalation flow that allows the exhaled air flow to be used again during inhalation. The rebreather 200 may comprise a non-pressurized oxygen generator, that may be activated chemically by mixing chemicals or using a special ignitable oxygen producing candles. With oxygen generators, the operating time for the re-breathing system could be extended and a small volume of oxygen is added into the rebreathing circuit keeping the total breathing volume constant. The rebreather 200 may comprise a counter lung connected to a carbon dioxide scrubber. The rebreather 200 may comprise a pressurized oxygen source connected to the port of the valve housing 214.

The person skilled in the art realizes that the present invention by no means is limited to the preferred embodiments described above. On the contrary, many modifications and variations are possible within the scope of the appended claims. For example, the filter housing 110 and the face piece 130 may have different shapes, dimensions and/or sizes than those depicted/described.

## Claims

1. A breathing mask (100), comprising
at least one filter (120) configured to remove contaminants from gas passing through the at least one filter,
a filter housing (110) arranged to house the at least one filter, and
a face piece (130) for arrangement on a user's face, wherein the face piece at least partially encloses the filter housing,
wherein the filter housing comprises a port (112) oppositely arranged the face piece, and at least one aperture (114) opening into the face piece, wherein the filter housing allows for gas to pass between the port and the at least one aperture through the at least one filter.

2. The breathing mask according to claim 1, wherein the filter housing is releasably attached to the face piece.

3. The breathing mask according to claim 1 or 2, wherein an opening (135) of the face piece is at least partially flexible and the port of the filter housing is rigid, and wherein the filter housing is releasably attached to the face piece by the port of the filter housing being fittingly inserted into the opening of the face piece.

4. The breathing mask according to any one of the preceding claims, wherein the at least one filter comprises a bio-filter.

5. The breathing mask according to any one of the preceding claims, wherein the face piece comprises a cushion part (137) arranged to come in contact with a user's face.

6. The breathing mask according to claim 5, wherein the cushion part is inflatable, and wherein the face piece comprises a valve (139) arranged externally on the cushion part for inflation of the cushion part.

7. The breathing mask according to any one of the preceding claims, wherein the filter housing comprises a first part (310) and a second part (320), and wherein the first and second part are configured to hold the filter in place between them.

8. The breathing mask according to any one of the preceding claims, wherein the at least one aperture comprises a plurality of apertures.

9. The breathing mask according to any one of the preceding claims, further comprising a sealing ring (140) arranged between the port of the filter housing and the face piece.

10. A breathing device (200), comprising
a breathing mask according to any one of the preceding claims, and
a rebreather (210) for closed rebreathing, wherein the rebreather comprises a counter lung and a valve housing connected to the counter lung, wherein the port of the filter housing is releasably connectable to a port of the valve housing (214).

11. The breathing device of claim 10, wherein the face piece of the breathing mask is arranged to at least partially enclose the port of the filter housing and the port of the valve housing in a sealing manner.

12. The breathing device according to claim 10 or 11, wherein the breathing device comprises a coupling mechanism (230) configured to couple the port of the filter housing and the port of the valve housing.
